# EUROPEAN PATENT APPLICATION

(11) **EP 4 817 525 A1**
(43) Date of publication of application: **07.10.2026**
(21) Application number: 25167500.5
(22) Date of filing: 31.03.2025
(51) Int. Cl.: A61B 6/03, A61B 6/00

(54) **SYSTEM AND METHOD FOR ACHIEVING LOCAL ISOTROPY HIGH-DEFINITION COMPUTED TOMOGRAPHY**

(71) Applicant: Koninklijke Philips N.V., 5656 AE Eindhoven (NL)
(72) Inventor: BONTUS, Claas, Eindhoven (NL); DAERR, Heiner, Eindhoven (NL); REUSCH, Tobias, Eindhoven (NL); STRIKER, Timothy, Eindhoven (NL); ROKNI, Michal, Eindhoven (NL)
(74) Representative: Philips Intellectual Property & Standards

(57) **Abstract**

System and related methods for facilitating operation of a rotational tomographic X-ray imaging apparatus (IA), comprising a user input interface (USI) capable of allowing a user of the imaging apparatus to specify desired one or more imaging requirements as user input. An adjuster (AD) is configured to adjust, based on the user input, one or more imaging parameters in respect of the imaging apparatus so as to improve isotropy of spatial resolution in tomographic imagery obtainable by operation of the imaging apparatus (IA) at the adjusted parameters.

## Description

### FIELD OF THE INVENTION

The invention relates to a system for facilitating operation of a rotational tomographic X-ray imaging apparatus, to an imaging arrangement including such as system, to related methods, to a computer program element, and to a computer-readable medium.

### BACKGROUND OF THE INVENTION

Imaging, in particular X-ray imaging, is one of the most important tools in the arsenal of the modem medic. X-ray imaging allows obtaining, in a non-invasive manner, information about internal anatomical structure and constitution of a patient which can quickly inform diagnosis or treatment. In an ever-aging population, particular in the higher income world, a demand for imaging services has soared. Combined with this is the ever more sophisticated imaging equipment itself, such as higher resolution X-ray detectors with a pixel size in the region of *250 microns* or higher. Such high-resolution detector equipment allows acquiring high resolution imagery.

Whilst emergence of such high-resolution detector as such is welcome as it allows yet better diagnosis and gathering of intelligence, there are unintended consequences: it has been observed that, if not accounted for, certain effects may undermine usefulness of such high resolution imagery, in particular for radiological diagnostic practice. Specifically, hitherto underappreciated anisotropy effects in relation to image resolution has been observed in tomographic imagery reconstructable from projection data detectable by such higher resolution X-ray detector. Such resolutional anisotropy may be difficult for the practicing medical users to interpret, and may thus potentially lead to misreadings.

### SUMMARY OF THE INVENTION

There may therefore be a need for improvements in tomographic imaging, in particular for improving image quality of imagery.

An object of the present invention is achieved by the subject matter of the independent claims where further embodiments are incorporated in the dependent claims. It should be noted that the following described aspect of the invention equally applies to the related methods, the image arrangement, to the computer program element and to the computer readable medium.

According to a first aspect of the invention there is provided a system for facilitating operation of a rotational tomographic X-ray imaging apparatus, comprising:-
- a user input interface capable of allowing a user of the imaging apparatus to specify desired one or more imaging requirements as user input; and
- an adjuster configured to adjust, based on the user input, one or more imaging parameters in respect of the imaging apparatus so as to improve isotropy of spatial resolution in tomographic imagery obtainable by operation of the imaging apparatus at the adjusted parameters.

In embodiments, the adjuster is to so adjust one or more imaging parameters based on an isotropy measure.

In embodiments, the adjuster is to so adjust one or more imaging parameters such that the isotropy measure remains within an acceptability range.

In embodiments, the said isotropy measure is based on respective spatial resolution along plural reference directions in image domain.

In embodiments, the said isotropy measure is based on a ratio of such spatial resolutions, including one such spatial resolution along a radial direction in respect of an acquisition rotation of the imaging apparatus, and spatial resolution along a direction perpendicular to such radial direction.

The said operation of imaging apparatus may relate to acquisition of projection data and/or to reconstruction of the tomographic imagery from so acquired projection data.

In embodiments, the said imaging parameters so adjustable pertain to any one or more of: i) frame number per rotation, ii) rotation period, iii) filtering in projection domain or image domain, iv) focal spot size.

In embodiments, the said adjusting by adjuster is based on calibration data measured in relation to the, or a, similar imaging apparatus.

In embodiments, the system, if no such improvement in isotropy is achievable given the user input, to issue a notification.

In embodiments, the notification includes a suggestion for modified user input.

In embodiments, the user input interface to allow user issuing an acceptance signal for the suggested modified user input and/or related one or more imaging parameters.

In embodiments, the system, on receipt of such acceptance signal, or automatically, to forward such adjusted one or more imaging parameters to control interface circuitry of imaging apparatus to thereby cause applying the imaging parameters to one or more components of the imaging apparatus, thereby readying the imaging apparatus for imaging.

In embodiments, the imaging includes reconstructing a tomographic image based on projection data acquirable by the imaging apparatus at the adjusted imaging parameters.

In embodiments, the adjusted imaging parameters are per sub-image for imaging for respective sub-images, the sub-images combinable into the tomographic image.

In embodiments, the user input interface is configured to allow user initiate imaging.

In embodiments, the said one or more imaging requirements include one or more of: i) distance to iso-center, ii) level of spatial and/or temporal resolution.

The distance may relate to a distance in respect of the ROI to be reconstructed to the imaging apparatus's iso-center. This distance may be one of a centroid of ROI to the iso-center, or of distal point of ROI, etc. Some of the imaging request may be provided by user in qualitative terms, such low, medium, high etc.

In another aspect there is provided an imaging arrangement, including a system as per any one of the aspect or embodiments mentioned above, and including any one or more of: i) the imaging apparatus, ii) a display device on which the user interface is presented, iii) a storage medium on which is stored data on which the adjusting is based, iv) a reconstructor for reconstructing projection data acquirable at the imaging parameters into tomographic imagery.

In another aspect there is provided a method of facilitating operation of a rotational tomographic X-ray imaging apparatus, comprising:-
- receiving, via a user input interface user input including one or more imaging requirements in respect of an imaging operation of the imaging apparatus;
- adjusting, based on the user input, one or more imaging parameters in respect of the imaging apparatus so as to improve isotropy of spatial resolution in tomographic imagery obtainable by operation of the imaging apparatus at the adjusted parameters.

In another aspect there is provided a method of measuring and storing such calibration data mentioned above.

In another aspect there is provided a computer program element, which, when being executed by at least one processing system, is adapted to cause the processing system to perform any one of the above methods.

In another aspect there is provided at least one computer readable medium having stored thereon the program element, or having stored thereon the calibration data.

In analyzing image resolution in CT, two effects may be observed: inhomogeneity and local anisotropy. Inhomogeneity means that the resolution varies over the FOV (field of view). Local anisotropy, of main interest herein and a special case of inhomogeneity, describes the effect that spatial in-image resolution in reconstructed imagery can be different along different spatial directions, such as in radial and azimuthal directions. The latter means that - especially small objects - may experience blurring in an inhomogeneous manner.

Image resolution in computed tomography is primarily determined by several factors. These are - besides the native detector pixel size/density - the size and shape of the focal-spot, the jumping mode of the focal spot (single focal spot versus flying focal spot), and the number of projections measured during one gantry rotation and the processing of the data (in effect, a projection domain interpolation) within the image reconstruction (e.g. via filtered back-projection).

Targeting isotropic resolution may require that at least some of these factors are adjusted accordingly. The increasing resolution of modem CT (computed tomography) detectors due to the small pixel size - especially in photon-counting CT - puts some burden on the other factors like focal spot size and shape and the number of projections. For example, a smaller focal-spot leads to a higher temperature of the anode disk of the X-ray tube, than does a larger focal spot size at same tube voltage (kVp) and emission current (mA). And increasing the number of projections per rotation may require either new detector technology with shorter integration periods and higher bandwidth of the slip ring of the rotating gantry, or slower rotation period (time).

It has been found, that in order to address these concerns, some balance may need to be struck between an-isotropic resolution, or a reduced image resolution. What is proposed herein is a setup that allows operator (user of CT) to navigate different imaging parameter settings for finding good balances between any two or more of isotropy, spatial resolution, temporal resolution, and size of the region of interest, and/or other applicable imaging parameters.

Local anisotropy increases with increasing distance to the rotation axis in an UHD ("ultra high definition") CT scan, utilizing the full detector resolution, but with an insufficient high sampling along the gantry rotation. This means that for a small properly centered region of interest (ROI) close to the rotation/iso-center, there is no need for a compromise with respect to isotropy, spatial or temporal resolution. On the other hand, in lung imaging or other, one is often interested in the best possible resolution over a larger ROI, such as 400 mm, with some portions being 200 mm from isocenter. In such scenarios with larger off-(iso-)center distances, local anisotropy might be detrimental to image quality and may lead to a wrong diagnosis. The proposed setup addresses anisotropy, by providing user with the option to choose imaging parameters so as to improve resolutional isotropy for a given imaging application as per user's imaging requirements.

Interestingly, in some embodiments, in order to achieve such improvement in isotropy, the imaging parameters, such as in relation to certain filtering or other applicable parameter (eg, focal spot size), may need to be adjusted so as to in fact reduce resolution along at least one (such as a single one) of the said (at least two) reference directions.

For present purposes, the imaging parameters may pertain to aspect of acquisition and/or reconstruction. Thus, for present purposes, reconstruction functionality may be part of the imaging apparatus, although the reconstruction may be done on a computing system located away from the acquisition equipment of the imaging apparatus.

The proposed isotopy measure, used herein may be thought of as an objective function to guide adjustment of imaging parameters so that image information across the image is better comparable. Thanks to the proposed setup, resolution along different directions is more uniform, thus improving image quality in the sense that the chance of radiological misreadings are reduced, whilst still maintaining parts of the benefits of "high res" imaging.

The user specified imaging requirements may pertain/include some of the imaging parameters. Imaging parameters that are not as such included in the imaging requirements specified by user may also be referred to herein as "free" imaging parameter(s).

The proposed setup is applicable to all manners of tomographic X-ray imaging, such as diagnostic or therapeutic CT. The use of the proposed system and methods in photon-counting CT apparatus, such as for use in spectral imaging (material discriminative X-ray imaging) is envisaged herein in particular, but so are more standard CT machines that use purely energy integrating detector equipment and associated data acquisition circuitry.

*"user"* relates to a person, such as medical personnel or other, operating the imaging apparatus or overseeing the imaging procedure. In other words, the user is in general not the patient.

*"(imaged) object*/*region, etc.*" is used herein in the general sense to include animate "objects" such as a human or animal patient, or anatomic parts thereof but also includes inanimate objects such as an item of baggage in security checks or a product in non-destructive testing. However, the proposed system will be discussed herein with main reference to the medical field, so we will be referring to the "object" as "the patient" or a part of the patient, such as an anatomy or organ, or group of anatomies or organs of the patient.

The terms *"isotropy"*/*"anisotropy"* relate to spatial directional independence/dependence, respectively, of a quantity, specifically in the present context of spatial resolution. The terms *"isotropy"*/*"anisotropy"* are used herein so that one defines the other: thus, an increase in isotropy, which is what is wanted herein, corresponds to a reduction/decrease of anisotropy, and the other way around. Thus, any statement herein in relation to one of the terms can be rephrased into an equivalent statement in relation to the other term, and the description herein is to be construed accordingly. Thus, a reference to either *"isotropy"* or *"anisotropy"* herein is indirectly a reference to the other term, *"anisotropy"* or *"isotropy",* respectively, but in an opposing sense. At times one or the other term will be used herein, as seems appropriate in the context.

### BRIEF DESCRIPTION OF THE DRAWINGS

Exemplary embodiments of the invention will now be described with reference to the following drawings, which, unless stated otherwise, are not to scale, wherein:
Fig. 1 shows a schematic block diagram of a tomographic imaging arrangement as may be envisaged herein;
Fig. 2 illustrates tomographic imaging geometry, and anisotropic effects in tomographic imagery;
Fig. 3 illustrates imaging components of a tomographic imaging arrangement;
Fig. 4 shows a block diagram of a facilitator system for adjusting one or more imaging parameters; and
Fig. 5 shows a flow chart of a computer-implemented method of facilitating operation of a tomographic imaging arrangement, in particular for increasing in-image isotropy of resolution.

### DETAILED DESCRIPTION OF EMBODIMENTS

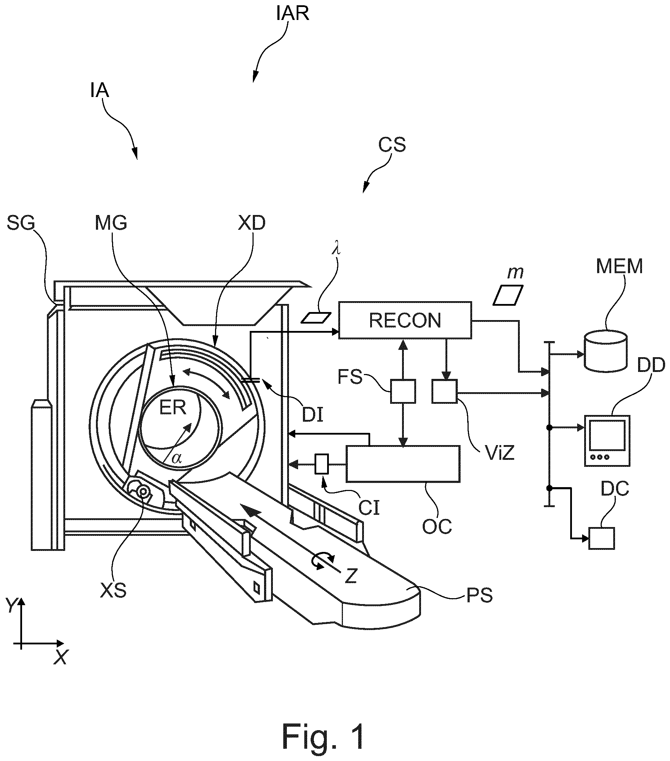

With reference to the block diagram of Fig. 1, this shows an imaging arrangement IAR as envisaged herein in same embodiments, and that may be used in the medical realm or other. The arrangement IAR broadly comprises an imaging apparatus IA ("imager" for brevity), and a computing system CS. The computing system CS may be coupled via a data transfer interface DI with the imager IA. The computing system CS may be part of the imaging apparatus IA. Computing system CS may receive, for processing, measurement data λ acquirable by the imager IA of a region of interest ROI. The measurement data λ may be particularly so processed into imagery *m* in relation to the ROI. What is mainly envisaged herein is that the imaging apparatus is X-ray based and is of the rotational tomographic type. Thus, the imagery *m* obtainable herein includes in particular sectional imagery in 3D of the region of interest, to better lay bare internal structures of interest for example.

Broadly, what is proposed herein is a novel type of computing functionality, referred to herein as the facilitator system FS, that allows more efficient operation of the imaging apparatus IA or of the imaging arrangement IAR more generally. The facilitator system FS may be part of the computing system CS or may be a standalone computing system suitably interfaced with the imaging apparatus IA or imaging arrangement more generally. In particular, the facilitator system FS may support in embodiments a user interface that allows a user controlling operation of the imaging apparatus in a manner so as to avoid, or at least reduce, certain undesirable in-image effects that may otherwise manifest, in particular when using high definition/resolution (*"high-def"* or *"high res"*) X-ray detector equipment with pixel size in the region of *200-250 microns* or more. In particular, such unwanted effects include inhomogeneity of spatial resolution across the obtainable reconstructed imagery *m.* Thus, spatial resolution may vary across the image which may be undesirable as will be illustrated below in more detail. Yet more particularly, certain unwanted anisotropy across the image in relation to the spatial resolution has been observed when using such high-res detectors for imaging. What is wanted instead is spatial resolution isotropy, so that the spatial resolution does not vary across the image. It should preferably be uniform, or should at least remain within a predefined acceptability range, across the imagery *m.* This can be achieved by operation of the facilitator systems FS that is operable to adjust certain imaging parameters to thereby facilitate achieving such (spatial) resolutional isotropy in tomographic imagery.

Before explaining the facilitator system FS in more detail, further details of general operation of the imaging arrangement are provided first for better context.

As mentioned, the imaging apparatus IA is preferably X-ray based to include an X-ray source XS from whose focal spot, an X-radiation beam emanates and passes through examination region ER and hence through the imaged object ROI. The radiation, in form of the X-radiation beam, interacts with matter that makes up ROI, and, as a consequence of this matter vs radiation interaction, is modified. This modification encodes spatial details of internal structure of object, thus allowing imaging. It is the so modified radiation that is detected during imager's operation by its X-ray sensitive detector XD, arranged opposite imager IA's X-ray source XS, with at least the ROI residing therein between, in the said examination region ER.

The measurement data λ, in general projection data, of the region of interest, is then made available via wired and/or wireless data connection to the computing system CS for the said processing. Whilst projection data in and of itself may be of interest herein, such as for radiography, not excluded herein, it is often computational processing of the projection data that is required first to obtain, for a given purpose, more apt imagery m. This may be the case for example in tomographic X-ray imaging, which is mainly envisaged herein in embodiments, and is illustrated in Fig. 1 at the example of a diagnostic CT scanner with its characteristic "doughnut" shape, an annulus, with its opening forming the examination region ER into which a patient support PS (couch, table, etc.) may extend, with ROI/patient PAT on it. Having said that, instead of such diagnostic type imagers, interventional imagers IA of the tomographic type, such as C-arm scanners, as may be used for image-guided therapy or other, are also envisaged herein.

In such tomographic arrangements, the measured projection data is processed by a computational reconstruction unit RECON which implements a tomographic reconstruction algorithm. This is a mathematical technique that allows converting the projection data (spatial data), acquired in multiple frames of ROI from multiple spatial directions α for one or more z positions along rotational axis *Z,* into sectional imagery *m.* The reconstructed sectional imagery *m* may include either a single section/slice, or more usual, plural such sections, representing an image (sub-)volume/slab of at least part of examination region ER, and hence of ROI in it. Such sectional imagery allows representing a cross-sectional view of ROI to lay bare internal structure of the ROI, now disencumbered from disturbances of intervening structures as in purely projection only based imaging (such as in radiographic modalities). The longitudinal axis is in general parallel to, or indeed coincidental with, imager's rotation/imaging axis Z that is run as, illustrated in Fig. 1, into the examination region ER, and through imager IA's isocenter.

This imagery *m* so reconstructed from the acquired projection data may be used for various purposes such as to inform diagnosis, therapy, radiation therapy planning or whatever the clinical task at hand. The reconstructed sectional imagery may be passed through wired and/or wireless connection, through a bus system or similar, to a memory MEM, volatile or non-volatile, on which it may be stored. Such non-volatile memory may include a Picture Archiving and Communication System (PACS) in which imagery is stored for later review in a radiological review session by a radiologist, who may then draw up their rad-report. The imagery may be visualized by visualizer VIZ that maps the imagery onto a color or a grey value palette and drives video circuitry to obtain a graphics display which visualizes imagery *m* on screen of display device DD. There may also be, instead of, or in addition to the above, other data consumer(s) DC, such as artificial intelligence based diagnostic aids for example that process the imagery *m* to localize, flag-up or otherwise indicate possibly suspicious structures that may warrant a closer look by the radiologist etc. Other manners of processing of or application contexts for, such imagery abound, all envisaged herein in embodiments.

The imaging arrangement IAR may include an operator console OC, arranged as one or more computing systems, through which operator/user can control operation of the imaging apparatus /imaging session, to so start, halt or otherwise manipulate acquisition of the projection data λ or effect reconstruction, displaying of imagery, etc. One or more actuators, such as server motors or others, may allow adjusting imaging geometry to ensure that the region of interest to be imaged is in the field of view of the imager.

A data link DI (an interface(s)), such as a slipring interface or wireless link or other, allows passing the acquired data λ from detector XD to the computing system CS for processing. The supporting computing system CS be implemented on one or more computing entities/nodes as mentioned such as a workstation that is communicatively coupled to the imaging apparatus, in particular via data output link interface DI and/or in whichever way.

The computing system may be either remotely located or located in the same examination room, may be integrated into the imager IA, as the case may be and as needed. Use of slipring interface datalink or wireless datalink DI allows providing data λ from a mechanically rotational system, such as is often used in tomographic imagers, to the outside for processing, such as by the supporting computing system CS or other.

The imager IA of the tomographic type is configured for multi-directional projection image acquisition. The imaging apparatus IA is thus capable of acquiring projection images/frames λ along different projection directions α relative to the examination region ER, and thus the ROI. Acquisition may be done in embodiments by a rotational system where at least the X-ray source XS is arranged in a moveable gantry MG. The movable gantry (and with it the X-ray source XS in embodiments) is rotatable in a stationary gantry SG around the examination region/bore ER in which the patient/ROI resides during imaging. Opposite the X-ray source in the movable gantry there is the X-ray detector XD which may rotate with the gantry and X-ray source around the examination region ER to realize the different projection directions α. Helical or step-and-shot imaging protocols are envisaged herein where there is a relative translational motion along the longitudinal axis Z, between X-ray source XS and the patient PAT. For example, the patient support PS may be advanced through the examination region ER during the multi-directional projection image acquisition, for example during rotation of the X-ray source XS about patient PAT.

The CT scanner setup as illustrated in Fig. 1 is merely according to one embodiment, and other tomographic imaging equipment such as C-arm or U-arm scanners, cone beam CT arrangements, mammographic imagers, etc, are not excluded herein. The multi-directional acquisition capability may not necessarily result from a mechanical rotational system such as shown in Fig. 1. Non-(mechanical) "virtual" rotational imaging systems are also envisaged, such as in CT scanners of the fourth or higher generation, where multiple X-ray sources are arranged around the examination region in a source annulus for example. In addition, or instead, the detector XD may be arranged as a detector annulus around the examination region. Thus, in such systems there is no mechanical rotation of the X-ray source XS or detector XD, or of both, but by switching source/detector in sequence, a virtual rotation is effected, which also allows multi-directional imaging.

The projection data λ (and the imagery *m* reconstructable therefrom) may be provided in digital form/format, that is, as a set of spatially resolved numbers. The detector XD may be configured to acquire the projection data λ in such digital form natively. A flat panel digital detector module XD may be preferred herein. Such detectors comprise a radiation sensitive imaging plane, or more generally an imaging surface, as curved detectors are also envisaged. The imaging surface is made up of radiation sensitive pixels which are operative to resolve the impinging radiation, after its passage through the ROI in the examination region, into the spatially resolved set of numbers. Such spatial digital data can be made available by detector XD on read-out as a matrix or tensor. Specifically, detector-sided data acquisition circuitry (DAQ), partly or wholly included in the X-ray detector XD, may be operable to process, in particular, convert electrical charges that accumulate in the detector XD due to exposure of the imaging surface to the X-ray beam, into digital values which can then be processed as described. Yet more specifically, the detector XD may include operational control circuitry (not shown) and read-out circuitry, arranged for example at and along the sides of a pixel matrix of the detector. Such circuitry allows reading out (electrical) charges accumulated at transistors that are used to form the respective pixels. The said charges λ' are due to X-ray exposure. Controlled by control circuitry, individual pixel positions in the detector matrix can be read out by drain lines, along which a respective charge is collected, integrated over time by integrator circuitry) and may then be output ("read-out") by read-out circuitry. Such read-out projection data λ can be output through the data link DI for processing by system CS, or by any other data consumer(s) DC, as above for tomographic reconstruction and/or otherwise.

The following spatial 3D co-ordinate system as illustrated in Fig. 1 may be helpful in the following. There is the said imaging/rotational Z axis that extends through the imager's bore ER, and preferably through the isocenter. In a plane perpendicular to the rotation axis Z, the other two axis X and Y span the tomographic imaging planes as indicated to the left of Fig. 1.

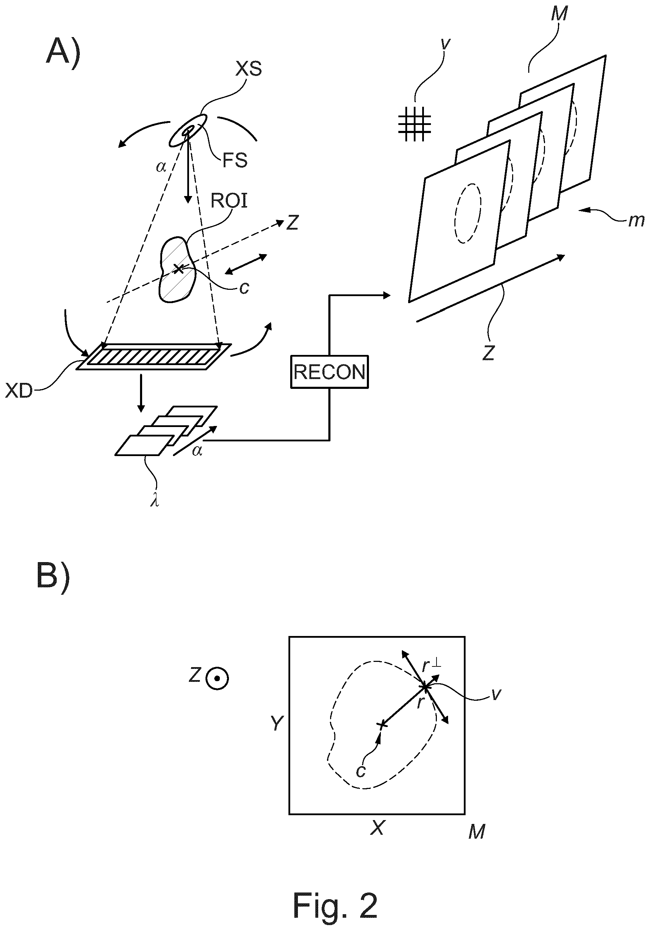

Turning now to the facilitator system FS in more detail, its principle of operation is illustrated with reference to Fig. 2.

Specifically, and referring first to Fig. 2A on top, this Figure illustrates obtaining of tomographic imagery m as envisaged herein in embodiments. Broadly, obtaining such tomographic imagery m (that is, operation of imager IA) can be conceptualized as a data processing chain, made up of two parts: a principally hardware based part shown to the left of Fig. 2A, and a principally computational/software part, shown at the center right of the Figure. Specifically, acquisition equipment of the imaging apparatus, in particular including the X-ray detector XD and source XS, are controlled by certain imaging parameters to acquire projection raw data λ at different directions α of the region of interest. To this end, and as illustrated by the curved arrows, acquisition equipment XD, XS rotates about rotation axis Z and hence about the region of interest. The rotation axis Z passes through iso-center c in between X-ray source XS and X-ray detector XD. Ideally, the region of interest is centered on the iso-center and hence the rotation direction Z passes therethrough, although the region of interest may extend in plural directions away from the iso-center, and thus the rotation axis, so that parts of the region of interest are further away from the rotation axis Z/iso-center c than are others. The X-ray beam issues forth from a focal spot FS of the X-ray source XS. Size of the focal sport FS may be adjustable, such size being one of the adjustable imaging parameters as envisaged herein.

Once the projection data λ measurements is acquired, this is processed computationally by the reconstruction component RECON as mentioned earlier. Reconstruction component RECON implements said tomographic reconstruction algorithm to compute the tomographic imagery *m* in image domain. Image domain *M* is a voxelized 3D space. Such voxelization is via spatial grid of voxel points v. The imagery *m* is located in such voxelized space and can be thought of as comprising multiple sections through the region of interest at different positions along the Z axis. Some of the imaging parameter(s) may relate to algorithmic particulars of the reconstruction algorithm, such as the type of filtering used, etc. Having said that, some filtering may also be done instead, or in addition, in projection domain, on the projection data.

The ultimate spatial resolution of the obtained tomographic image *m* depends on the native resolution (pixel numbers /density, size of pixels) of the detector, but also on other adjustable imaging parameters.

Thus, operation of the imager IA, or of the imaging arrangement more generally, is controlled by specifying said imaging parameters. The imaging parameters can pertain either to the manner of acquisition of the projection data (left hand side of Fig. 2A), or to the computational processing by reconstruction or other computational steps on the right of the Figure. Thus, the imaging parameter may pertain to the projection domain and/or to the image domain *M* as will be explained in more detail below. Thus, some of the (projection domain) imaging parameters may relate to how the projection data is acquired and/or certain conditioning (filtering) thereof, whilst other imaging parameters (the image domain parameters), may relate to the manner in which the projection measured data λ is to be processed into the wanted tomographic imagery *m.* To the extent that the adjusting of the parameters relates to the projection domain parameters, this can be done prior acquisition. To the extent the imaging parameters relate to image domain, adjustment can be done post-acquisition or can still be set pre-acquisition. Thus, the facilitator FS may be operable prior- or post-acquisition, or partly both, as needed. Preferably, operation prior acquisition is envisaged in the main (but not only).

Turning now to Fig. 2B below, this illustrates the mentioned image quality deficiency, in that there may be high resolution of image thanks to the high res detector, but only in parts of the image. Specifically, such spatial in-image resolution may vary differently along different directions in image domain *M.* In other words, there may be a certain amount of anisotropy manifest in imagery *m* obtainable in above setting 2A, in particular when high-res detector equipment XD is used for acquisition. Thus, it has been observed herein that such spatial resolution anisotropy ("*(spatial) resolutional anisotropy")* in tomographic imagery is coming to the fore more palpably when such high res detector is used. In earlier set-ups with lower resolution detectors this effect was not appreciable. It has been found that resolutional anisotropy can reduce the diagnostic value of the imagery. For example, it has been demonstrated by imaging ring shaped test objects, that resolution of such objects further away from the iso-center exhibits bogus variations in grey values. This effect, which may be referred to herein as anisotropic blurring, may for example coax the diagnostician/ radiologist into believing that there is a deficiency of density of matter, although, in reality, there is not. For example, such anisotropic blurring may lead to erroneous diagnosis of bone shrinkage. Such erroneous finding may be detrimental to the patient, and may also lead to inefficient use of medical resources, unnecessary follow-ups, for example, further imaging may be requested, potentially all in vain.

Image resolution in general, in particular spatial resolution, pertains to the ability of the imaging system to resolve details structurally in image domain. In distinction, there is also the concept of temporal resolution which pertains to the ability of temporal sampling, in order to resolve over time events (motion or deformation, such as in cardiac imaging). Such events may be recordable in imagery sampled over a certain period of time, as is the case for tomographic imaging, where the detector rotates around the region of interest and acquires at times during the rotation, respective frames temporarily apart along the acquisition path. Temporal resolution is a function of the rotation period/frequency with which the detector/source rotates around the region of interest, and the integration time with which the detector can read-out individual projection frames. In CT imaging the temporal resolution of the system should be faster than the time scale on which the object motions occurs to be able to acquire the frames with only a small amount of motion during one rotation.

It is spatial resolution, and its anisotropy, that is of main interest herein although it has been found that aspects of temporal resolution, in particular when set by the user as an image requirement, does have a bearing on spatial resolution and its anisotropy.

Turning now to spatial resolution ρ in more detail, this can be measured by a modulation transfer function (MTF). The MTF is a good approximation of the optical transfer function (OTF), if phase modification measurably by the phase transfer function (PTF) are negligible, as they are in the present X-ray based context.

Spatial resolution can be measured as the ability to resolve a test line pattern of a test object. The object may be made of a grid of radiation opaque bars ("lines") with voids (gaps) between any two neighboring bars. The distance between bars becomes smaller in a direction across the bars. Test imagery reconstructed from acquired projections over those barred objects can be analyzed to estimate spatial resolution ρ. In particular, contrast modulation/contrast depth can be measured in terms of local minimum and maximum intensities as recorded in the test imagery. The intensities may be coded as HU (Hounsfield unit) grey values (with black and white values as extremes for the voids and the bars, respectively), or in whichever encoding used. Based on such image value intensity analysis, an approximation of the MTF, and hence of the resolution p, may be obtained. An inverse spatial Fourier transform may be used for this as understood by those schooled in the calibration art of intensity-based imaging systems. Resolution *ρ* may be specified as the line pattern in units of line pairs per unit length (eg*, cm* or *mm,* etc) at which the MTF drops to a certain percentage s% value , such as *s=10* or other. The percentage s% describes the response measurement relative to a fully resolved measurement. Typical values reported are the line patterns for 80%, 50%, and 10%.

Spatial resolution *ρ* can be associated with each voxel point v, and a direction perpendicular across the bars. As said., resolution *ρ* is measured in terms of "line" (bar) pairs *("lp")* per unit length, such as per millimeter *mm or cm ("lpm"),* for a given percentage *s*% value of the MTF taken along such reference direction in image domain. Thus, spatial resolution is a directional quantity which can be likened to vector field where each voxel position v is assigned such resolution *ρ= ρj* along a certain spatial direction *j*.

With continued, but now more detailed reference to Fig. 2B, this illustrates anisotropy as may be encountered in tomographic imaging using in particular high res detectors. In particular, the resolution differs with distance from the iso-center c as illustrated in the Figure for an arbitrary voxel position v. On top of this variation, there is also a local anisotropy at the voxel position as illustrated by the two reference directions *r* and *r*^{⊥} "*r perp"* . Direction *r* may pertain to the radial distance or direction from the iso-center c to the voxel position, whilst *r*^{⊥}, the tangential or azimuthal direction, is perpendicular to direction *r*.

It has been found that the resolutions *ρᵣ*, *ρ*_{*r*^{⊥}} along respective directions *r*^{⊥}and *r are* different for most or each voxel positions, with this local effect more pronounced the further away, that is the longer distance *r*, is from the iso-center c. What is proposed herein in one aspect is a measure τ that measures this local anisotropy. It has been found that such measure τ is a function of the imaging parameters *p* used. What is proposed herein is to adjust the imaging parameters, in a manner so that the isotropy measure τ (or equivalently the anisotropy measure (depending which way it is measured)) remains in an acceptability range. Such measure has been found to be a ratio τ = *ρᵣ* / *ρ*_{*r*^{⊥}} , or a function *F* thereof, τ = *F*(*pᵣ*/ *ρ*_{*r*^{⊥}}). The acceptability range of the ratio of resolutions along different directions per point v may be expected be around unity for a balanced local isotropy behavior, which may be preferred herein.

The user may specify, eg through a user interface, one or more imaging requirements *q.* Some of these imaging requirements *q* may specify certain imaging parameters *p* or are functions of such imaging parameters. Imaging parameters corresponding to the imaging requirements may be held fixed, whilst other imaging parameters, referred to herein as free imaging parameters *p*', are allowed to vary so as to bring about a variation in the associated isotropy measure τ. It is an objective herein to decrease (local) anisotropy (increase local isotropy), wherein such decrease may be defined in terms of thresholding, or, more generally, by the said acceptability range. Thus, for example, parameters *p* may be adjusted to that τ = *F*(*pᵣ*/ *ρ*_{*r*^{⊥}}) ≈ 1. *F* may be the identity function.

The isotropy measure $τ : {ℝ}^{n} - > ℝ$ is a *n*-dimensional ( $n ∈ ℕ$) scalar function that maps the imaging parameters *p* into the reals (a number). Thus, the isotropy measure τ may be a number, such as may be based on the above ratio. The imaging parameters can be conceptualized, processed and stored, after suitable encoding/embedding, as points in a vector space ${ℝ}^{n}$. The imaging parameter space ${ℝ}^{n}$ can be high-dimensional, depending on the number of parameters one wishes to consider.

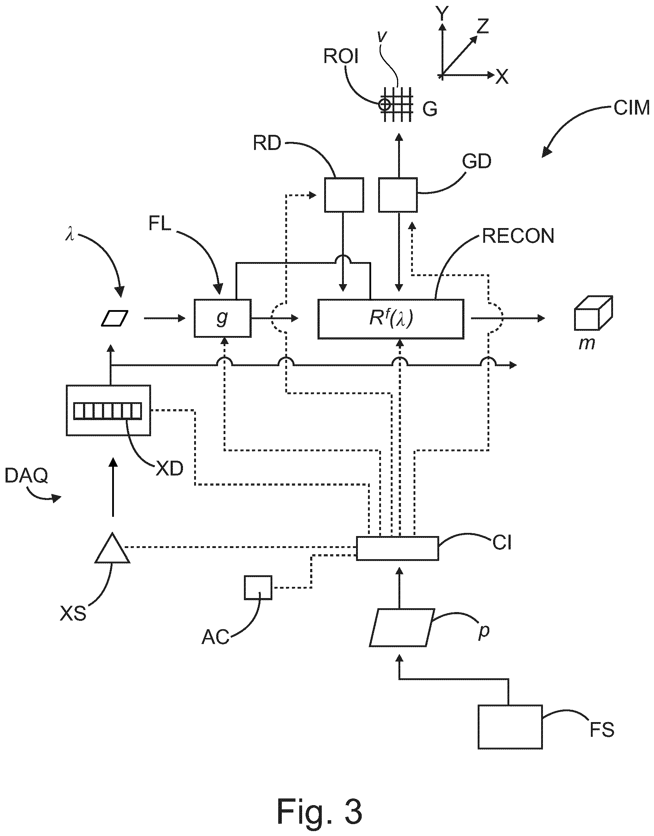

Imaging parameters p, and how they relate to certain imaging components CIM of the imaging apparatus IA, or imaging arrangement IAR more generally, are illustrated in Fig. 3, to which reference is now made.

In more detail, Fig. 3 shows possibly manifold components CIM that need to be provided with imaging settings as per imaging parameters *p* (such as instructions. etc). Imaging facilitator FS provides such imaging parameters *p*. These are propagated through control interface circuitry CI, including suitable interfacing API's, to the various components CIM of the medical imaging apparatus IA.

The imaging controlling components CIM of imager IA may include the X-ray source XS, the X-ray detector XD. More generally, such components may be members of the data acquisition component operable to acquire the measurements λ. In addition, downstream to acquisition, the acquired measurements λ may be processed, such as by a reconstruction algorithm RECON, to compute tomographic or volumetric imagery *m* in image domain.

As further illustrated in Fig. 3, the components CIM may include one or more actuators AC that allow changing the imaging geometry, in particular changing collimation, pitch (number or rotation of detector XD/source XS per length unit along which patient table PS (and hence ROI) is advanced along *Z,* etc.

In addition, some parameters may relate to the energizing of the X-ray source XS, such as an X-ray tube. Such parameters may relate to voltage and/or amperage of tube XS. For example, some of the parameter(s) may describe the voltage across the tube's cathode and anode. In addition, or instead, the amperage of the anode may be changed that controls how many electrons are released, whilst the tube voltage determines the acceleration and thus the energy of the X-ray beam issuing forth from the focal spot FS when the electrons impact the source XS's anode at the focal spot FS.

Another one of the parameters may relate to the tube XS's focal spot size, and/or pulse time, etc. Thus, some X-ray tube XS may include an electron beam shaper (not shown). This may be operative to shape the electrons that bubbled off from cathode into an electron beam for better focusing on anode of tube XS. The electron beam shaper may be configured from higher density material as claws or similar to form an aperture through which some of the electrons escape to form the electron beam. In other embodiments, the electron beam shaper is a grid, which is electrified with a negative charge, to force, thus form, the electrons into the electron beam. In yet other embodiments, the electron beam shaper may include two or more electromagnet configurations that operate to generate a magnetic field through which the electrons pass, thus forming them into the said electron beam. The strength of the current through the emitter coil CL and the strength of the accelerator voltage, may determine the energy of the photons that make up the X-ray beam. A larger accelerating voltage may cause a beam of higher energy, as may be called for by certain protocols, in particular when imaging a thicker object. The intensity of the X-ray beam is controlled by the current at the cathode. Otherwise, a low signal to noise ratio will result as too few photons may be able to pass through the imaged objection (e.g. patient tissue) to reach the detector XD, due to their being attenuated by the object and due to scattering or other matter vs radiation interaction effects.

Some of the adjustable imaging parameter(s) *p* may also relate to certain parameters of a filter component FL, or to parameters such as regularizer parameters, or other parameters of the reconstruction algorithm RECON.

The reconstruction may use a filter *f* in the reconstruction process applied to image values in image domain (filtered back-projection). Alternatively, or in addition, the filter component FL may control filter operation g in projection domain applied to the projection data prior to reconstruction.

Specifically, the optionally filtered projection data is processed by the reconstruction component RECON into reconstructed imagery in image domain *M*. The reconstruction component RECON may implement the tomographic reconstruction algorithm. The reconstructor component RECON may implement the one or more reconstruction algorithms to process the projection imagery into imagery *m* in image domain. Specifically, the reconstructor RECON may compute the output image *m* as sectional imagery of the examination region (with the patient in it) for diagnostic, therapeutic or other purposes. The reconstructor RECON may be able to produce sectional volumetric image data *m.* However, this does not exclude producing a single image slice in the examination region as required. Thus, the reconstructed imagery may include the whole volume ER, a partial volume thereof, or a specific section therethrough. Volumetric reconstruction may be facilitated by helical movement, and/or the 2D layout, of the X-ray detector XD. Any suitable reconstruction algorithm may be used by reconstructor RECON component, such as iterative, algebraic, statistical or analytical. Analytical reconstruction algorithm may include filtered back-projection (FBP). Iterative reconstruction algorithms are also envisaged in preferred embodiments. The examination region ER, or image domain *M*, is a portion of 3D space. Conceptually, image domain *M* can be thought of as made up of a spatial grid of the spatial points referred to above as voxels v. Such voxels v help define tomographic sectional imagery. The reconstructor RECON applies the reconstruction algorithm to the projection data λ to populate the grid of voxels with image values, to so build up the volumetric sectional image *m* in the image domain *M.* The imaging domain is in distinction to the projection domain. The projection domain is defined by the radiation sensitive pixel matrix of detector XD. Thus, reconstructor RECON may be understood as a mapping from projection domain into image domain, *R^{f ,g}*(*λ*) *=* m , with fg being the optional image domain filter and/or projection domain g filtering.

An optional region of interest definer RD relates to an imaging geometry defining component. As mentioned, such may effect mechanical movement of the gantry for example, and thus of the source XS and/or the detector XD, and/or may effect motion control of the patient support PS, such as in CT. Another imaging geometry defining component may include a collimator in X-ray. Thus, some parameter(s) *p* may relate to positions of radiopaque collimator blades of collimator.

A grid definer GD defines the mentioned spacing of the spatial grid and voxels in an image co-ordinate system of the examination region (*X,Y,Z*). An aspect of resolution in image domain *M* for example may be set in this manner, based on one of the imaging parameters.

Any one of the above mentioned components in Fig. 3, such as the filter component FL, the ROI definer RD, the grid definer GD, the reconstruction component RECON, etc., may be arranged as separate, discrete, components as shown in Fig. 3. However, this may not necessarily be the case in all embodiments, as the above, "spread-out", layout in Fig. 3 is largely driven by clarity of presentation. Thus, in other embodiments, some or all of the components may be coalesced into fewer components by integration or combination, or even into one single component. For example, the filter element component FL is sometimes part of the reconstruction algorithm implemented by the reconstruction component RECON.

Also, it will be appreciated that the above list of parameters and components CIM are merely illustrative and non-exhaustive, and, depending on the imaging task, some components may be used, whilst others are not. Thus, some of the components above may be optional.

The imaging requirements q as far as acquisition is concerned, may relate to any one or more of the following imaging parameters: resolution (may be relate to focal spot size indirectly by user qualitatively choosing a resolution level), rotation period, acquisition mode (axial or helical including the pitch factor), focal spot jumping mode, etc. In terms of imaging requirements q as far as post-acquisition (in particular reconstruction) is concerned, such may relate to any one or more of recon FOV (in particular size and/or distance from iso-center of ROI), matrix size a per grid definer GD, reconstruction increment along Z, (reconstruction) filter *f* and/or g, or others.

The free parameters *p'* that may be adjusted herein to improve resolution isotropy may include any one or more of: acquisition imaging parameters such as number of views (number of projection frames per rotation of source/detector), focal spot size, projection data filtering g such as binning of measurements from different pixels, and focal spot jumping mode. In terms of post-acquisition imaging parameters, filtering *f* of reconstructor may be adjusted.

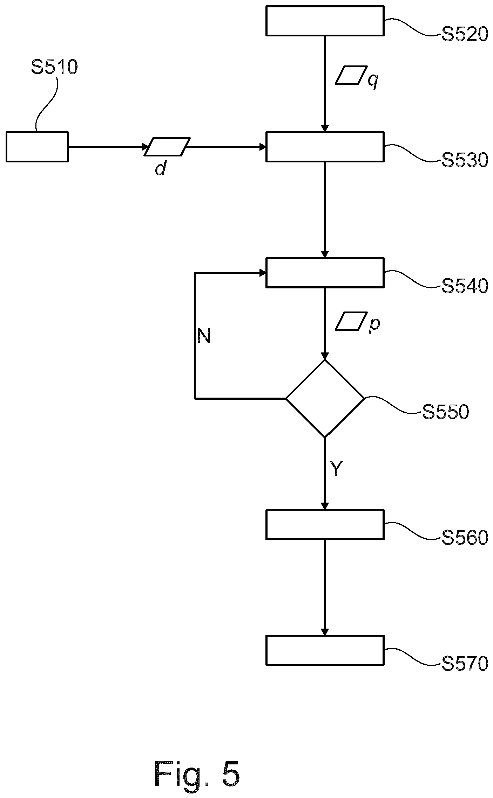

The resolution as specified by the user in the imaging requirement(s) q may be so specified qualitatively, such as *"standard resolution", "high resolution"* and *"ultra high resolution (UHR)"* or similar, which can then be mapped to adjustments *p* in relation to the focal spot size selection. For example, for an imaging requirement *q* for UHR with a smaller FOV, a larger focal spot may be required, and lower number of views. However, an imaging requirement *q* for UHR with a smaller FOV may call for a smaller focal spot, with a higher (eg, max) number of views possible due to a selected rotation time. Other scenarios will be described below at Fig. 5.

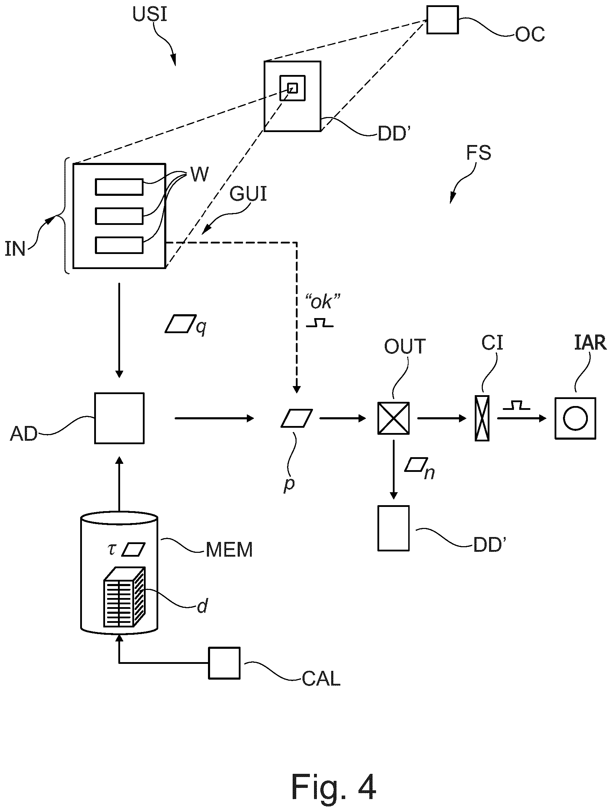

Reference is now made to Fig. 4 which shows a schematic block diagram of functional components of the facilitator system FS. The facilitator system FS may be run on one or more computational nodes, such as a workstation and/or the operator console of the imaging apparatus, or any other suitably computing system, connectable in a data communication structure with other components of the imaging arrangements. The facilitator system FS may support a user interface USI which may be part of the operator console, and/or of the workstation post acquisition, as needed or may be arranged at both. In particular, the user interface USI may be arranged as a graphical user interface ("GUI") as illustrated in upper left of Fig. 4.

The graphical user interface USI may then be visualized as a graphical user interface screen on a display device DD'. The display device DD' may be onboard display device the operator console OC, or may be another display device such as used for viewing the imagery *m.* The graphical user interface USI may include graphical user interface elements (widgets) *w* that allow the user to specify image requirements *q.* Thus, the user interface represents an input interface IN for inputting the imaging requirements *q,* and pass them on to an adjuster component AD ("adjuster"). Based on *q* , adjuster AD adjusts imaging parameters *p,* or at least attempts to so adjust, so that the isotropy *τ* = *τ(p*|*q),* which is a function of the parameters *p* and requirements *q,* remain within acceptability range.

As mentioned, some of the imaging requirements *q* may pertain to a sub-set of imaging parameters *p, p* ∩ *q* ≠ Ø. The parameters that pertain to imaging requirements *q* are to be held fixed as desired/specified by the user, whilst the other (free) parameters *p'* can be adjusted by the adjuster AD, using the anisotropy function τ as an objective function to remain with an acceptability region. If such acceptable parameters *p'* that allow maintaining the anisotropy function τ in the acceptability range are found, these may be output at output port/interface OUT. This set of adjusted parameter(s) *p'* may be displayed on display device DD', or on any other display device, for quality check by user. If found acceptable, the user may issue via user interface USI or in any other way an "OK" signal, and in response, the adjusted parameters p' may be forwarded to imaging apparatus, and applied there to respective imaging components CIM through suitable control interfacing CI. Imaging can then commence on user request or automatically to acquire the projection data at the adjusted imaging parameters, thereby leading to tomographic imagery of higher quality that is, of higher isotropy, or full isotropy, as needed.

If the image requirements are incompatible with the requirement for the requested level of isotropy, a notification *n* may be issued to this effect. Such notification *n* may be brought to user's attention, such as through displaying on the display device DD', or on any other display device, and/or by sounding out, or by issuing an alert signal in whichever manner. The user can then review or readjust their imaging requirements or can ask the adjuster AD to suggest modified requirements *q'*, which can then be accepted by the user or not. The modified requirements *q',* and related parameters, may be computed by the adjuster so that the requisite isotropy level as per acceptability range can be met. In other embodiments, on finding of such incompatibility, the modified requirements *q'* are presented to user as suggestion automatically, without need for the user explicitly asking for this.

Operation of the adjuster AD may be based on calibration data d acquired by a calibration system CAL. The calibration system CAL may be implemented as a computing system that runs a test script to control operation of the imaging apparatus. According to the test script, imaging apparatus IA is used to acquiring test imagery at different imaging parameters. The imaging parameters are automatically varied, and for each variation test corresponding imagery is acquired. The test imagery so acquired may be of the above described test object having structure of a grid of radiation-opaque bars at different pitches. The different imaging parameters are stored in association with isotropy measure τ as measured in the acquired test imagery corresponding to each imaging parameter set. In the test script, the various imaging parameters are stepped through at a suitable step width or increments. At some or each step, the test bar object is imaged, and the isotropy measure is recorded either at each voxel v' or at reference positions. Thus, the test data *d* may be stored as high dimensional data structure, such as tensor, where each entry corresponds to an isotopy measured d={ *τ*(v') -> *p= (p1, ... , pN)* }. The adjuster AD can use data *d* as a look-up table, to find for each given subset of parameters as prescribed per *q*, the associated imaging parameters *p'* that yield acceptable τ'*s* with *τ(p'*|*q)* ∈ *Iₐ*. Other interpolation for the *p* 's may be considered, be it multi-linear or polynomial. Techniques from solving for system of non-linear equations/inequalities may be used such as Broyden, Newton, finite difference methods, etc.

As to the resolutional isotropy measure, the earlier mentioned ratio of resolutions along plural reference directions have been found to yield good results, although this is not at the exclusion of other such measure definitions. Thus, with continued reference to Fig. 3, resolutions *ρ* along the respective reference directions, such as *r, r-perp,* may be used to form ratios τ = τ(v) = ρᵣ/ρ_{r^{⊥}} (or its inverse of function thereof) as a measure for local isotropy at location v, with *r, r-perp* refereeing to the particular location v considered in image domain *M*. A value of this measure *τ* = *τ*(*v*) = *ρᵣ*/*p*_{*r*^{⊥}} ≈ 1 of unity would indicate good local isotropy. This definition has been found herein to yield a useful, because robust and reliable, measure for such isotropy. This definition admits to various modifications and extensions, all envisaged herein in embodiments. For example, instead of ratio *τ* = *τ(v)* = *ρᵣ*/*p*_{*r*^{⊥}} the difference might be used with *τ* = *τ(v)* = |*ρᵣ-ρ*_{*r*_{⊥}}| ≈ 0 indicating good local isotropy. In addition, instead of using a bi-directional definition along *r, r*^{⊥}, any other two spatial directions may be used instead herein, and so can an additional direction dimension, with a spatial directional component along direction *Z* into the image plane. For example, a tri-directional definition may be used, based on respective resolutions *ρᵢ, ρⱼ, ρₖ,* and these can then be combined with the other two resolutions, such as forming a product or any other functional composition to construct a suitable tomography measure with factors, such as, for example: *τ* = *ρᵢ*/*pⱼ * pᵢ*/*pₖ * pⱼlpₖ.* Alternatively, any two of the factors may be used, with *i,j,k* referring to any three non-coplanar spatial directions in M, not necessarily confined to *X,Y* and *Z*. However, measuring bi-directionally that is, along two spatial directions, has been found herein to be of sufficient performance.

The user provided imaging requirements q as envisaged herein, include any one, or both of, in particular, the distance *r* of the region of interest ROI (e.g. maximal recon FOV) from the iso-center *c*, and a desired level of spatial resolution, in particular in a predefined or user defined neighborhood *U(r)* at the chosen distance away from the iso-center. The resolution level can be merely specified by the user qualitatively, such as *"high", "low", "medium",* etc. These qualitative levels can be mapped to respective resolution values ranges. In addition, or instead, the temporal resolution, such as the minimum rotation period (the amount of time needed for a full rotation of source XS/detector XD about iso-center) may be set by the user as such an imaging requirement q.

The distance *r* to the iso-center may pertain to a mean distance from the region of interest if off-center, distance from center c to ROI center or barycenter, or any other, or may pertain to ROI's furthest point from the iso-center c, as needed. For specifying such object(ROI)-to-iso-center distance, the user interface USI may support a visual representation of the region of interest. Such visual representation may be based on prior imagery, such as a scout image, in which the user can specify a point, or multiple points, spatially, to so indicate the distance or distances of interest at which the requested spatial resolution is sought for.

GUI input elements w that allow user to indicate the imaging requirement, may include appropriate GUI widgetry, such as textbox(es), menu function (e.g., of the drop-down type, or other), spinners, sliders or any other suitable widgetry that allows the user to adjust the imaging requirements either continuously or in steps, including any one or more, or all of the three example requirements *q* mentioned above: spatial resolution, temporal resolution and distance to the iso-center. The user interface may be interactive, such as a GUI supported on an interactive screen portion of a touch screen, where user can specify the imaging requirements by touch screen action, stylus or computing mouse action, or in any other. Specification by keyboard entry is not excluded herein.

It will be understood that operation of adjuster AD may not necessarily result in the sought after local isotopy everywhere in image domain. Indeed, it may be sufficient herein to solve for suitable imaging parameters *p',* such that for all or a predefined number or proportion of *v*'s ∈ *M* in said neighborhood *U(r)* (e.g., a sphere, cube or other) at the specified distance from c, the isotropy measure *τ* remains within the acceptability region *Iₐ*. For example, using the ratio measure, the adjuster is operable so that *τ*≈ 1 for such *v*'s.. The said proportion of the v's that have the desired isotropy may be a design parameter or may be set by user via user interface USI.

Optionally, the user interface USI may include one or more elements w to adjust the acceptability region Ia. Optionally, the user interface USI may include element w to allow user to disable /enable facilitator FS. Thus, user may be able to specifically enable via an "isotropy booster" element w or similar, the above described functionality of facilitator FS, or user may choose to disable the functionality afforded by facilitator FS.

Reference is now made to the flow chart of Fig 5, which illustrates operation of the above mentioned facilitator system FS. Having said that, the below described steps may also be understood as one of a computing implemented method in its own right for facilitating operation of tomographic imaging apparatus, in particular of those using high def/res detectors, in order to ameliorate resolutional anisotropy.

At step S520, one or more imaging requirements q in terms of suitable parameters, are provided. This may be done through a user interface as described above. At least some of the imaging requirements q may be specified as higher level qualitative descriptions, such as in terms of a rating (such as "high", "middle", "low"). The imaging requirement may pertain to distance from iso-center c, desired spatial resolution there, and temporal resolution (temporal sampling over acquisition), or others, instead of, or in addition to, any one or more (e.g., all) of the foregoing.

At step S530 such imaging requirements q are received for computational processing as will now be described.

At step S540 free parameters p, other than the imaging parameters prescribed (either directly or indirectly) at step S520 via the *q*'s*,* are adjusted, so as to ensure that the functionally related isotropy measure τ remains within a pre-defined acceptability region, *τ(p'*|*q)* ∈ *Iₐ*.

Operation of this adjuster step S540 may be based on calibration data *d,* earlier provided by calibration step S510, and as explained above in relation to calibrator system CAL. The calibration data *d* may be collected and organized in the form of a multi-dimensional look-up table (LUT) that allows approximating e.g. interpolating, the function τ for the desired *p*'s. The interpolation may not necessarily be linear.

Adjuster step S540 may be based on any suitable algorithm in regard of the isotropy measure τ. In particular, the functional relationship *τ(p*|*g)* may be represented as an algorithm. For example, the functional relationship between τ and *(p,q)* may be modelled based on physical model(s), using insights from radiation physics, imaging geometry, detector specifications, and/or any other.

In embodiment, in the prior calibration step S510, applied imaging parameters and requirements can be sampled at a suitable step width, and the associated isotropy measure response τ is recorded. Based on this, in general high dimensional, set of points, the said (high dimensional) LUT can be obtained. This can be represented as a high dimensional tensor. With an efficient traversal thereof, given the q coordinates as per step S520, a check can be run through the LUT whether parameters *p'* can be found such that the resulting measurement *τ* remains within a user specified, or otherwise prescribed, acceptability region (an interval) *Iₐ, τ(p*|*q)* ∈ *Iₐ*. Thus, in general, whichever approach is used, adjuster step S540 provides as solution(s) the set of one or more parameters {*p'*| *τ(p'*|*q)* ∈ *Iₐ*}. Interpolation from the measured data *d* can be done via multi-linear interpolation, or interpolation via high-dim polynomials, such as Bezier, Spline, or Bernstein, etc., or any other. The adjuster step may be based on gradient methods or may be based on improving a cost/utility function in an optimization setup, etc.

As an alternative to such "analytically/classically" minded approaches, a machine learning ("ML") model can be used, where human expert *annotated p** values are associated with training imaging requirements *q*,* such that isotropy is achieved (the notation "*" indicates that the respective data is training data, so is different from data *p,q* used in ML-testing or -deployment). In this manner, a regression model can be trained that regresses post-training the requirements *q* into imaging parameters *p'* to ensure a requested level of isotropy. In particular, local isotropy may be measured by any one of the above described isotropy measures *τ*.

At step S550 it is then checked whether, given the adjusted parameters *p'*, the isotropy requirement *Iₐ* is met. If not, a further adjustment may be made, and it is repeatedly checked whether the isotropy requirements are met. If, after a pre-set number of steps or once other stopping condition is fulfilled, it turns out that the isotropy cannot be met given the current set of *q*'s, a notification to this effect is issued, such as warning signal, either as an audio signal, or visually/graphically, or in any other form. As an option, modified imaging requirements may be proposed and included in the notification and brough to user's attention for the user to reject or accept. Such modified imaging requirements *q*' may be obtained based on specifics of the algorithm that is run in S540 to adjust the imaging parameters. For example, such suggestions *q's* may be obtained based on examining of applicable gradient or cost function behavior, or by randomization, etc.

The proposed isotropy measure τ may be understood as an objective function that guides the adjusting of the imagining parameters p, whilst using the imaging requirements *q* as constraints that are to be met: {*p'*| *τ(p'*|*q)* ∈ *Iₐ*}.

At step S560, once an acceptable set of parameters *p'* that respect the isotropy requirement is met, this set of parameters *p*' may then be applied to the imaging apparatus. Respective imaging components can then be (re-)set accordingly, based on the *p'* 's. Before so applying, the adjusted parameters may be displayed to use for approval. Thus, in this user-in-the-loop setup, the adjusted imaging parameters are only applied on user approval, with user issuing as respective "ok" signal via user interface.

At step S570, either automatically, or upon user manually sanctioning (via (another) "ok" signal) the so adjusted imaging parameters, imaging can commence at step S570 to obtain isotropy improved resolution imaging, with better isotropy in the imagery.

The below describes various use scenarios and combinations of imaging parameters and user imaging requirements as may be envisaged herein in embodiments:-

### Adjusting p as the number views per rotation to match isotropy measure τ acceptability range:-

This scheme might be optimal for those applications requiring local isotropy over a larger region (large FOV), where temporal resolution is of secondary consideration. For this scheme, the number of views (projections taken per rotation) is increased such that local isotropy is obtained over the full ROI. In practice this might imply that the gantry rotation speed is reduced at the expense of lower temporal resolution. This is because there is in general a smallest possible read-out integration time period ("IP"), and it needs to be ensured that there is enough signal strength detectable in each reading.

Isotropy measure *τ* (e.g., the said ratio of radial *r* to tangential r^{⊥}MTF) acceptability range may be set in this scenario at between 0.8 to 1.2.

In this and other scenarios, ROI size to be imaged may be established based on a scout scan or the selected recon FOV is established to obtain the largest distance *r* from the iso-center c. Then, the number of views is adjusted needs such that *τ* is within the acceptability range. This can be calculated/measured once, as described in the calibration step above, and tabulated in LUT d (with interpolation if needed).

### Adjusting p as the detector binning g, given an isotropy measure τ acceptability range:-

In cases in which the best possible temporal resolution is required and the number of views per turn is restricted, a reduction of spatial resolution might be the only possibility for obtaining local isotropy.

In this scenario, detector binning g provides one way for achieving this. "Binning", a type of filtering, means that the number of detector pixels is reduced virtually by combination of readings from neighboring pixels. This can be achieved by application of a box car or a Gaussian filter kernel. Binning does not necessarily mean that the number of pixels is reduced by an integer ratio. In fact, spatial isotropy over the entire ROI at the minimum loss of resolution will in general be obtained if this ratio is fractional. In practice, one would therefore determine parameters for each scanner model and various scan modes describing the optimal ratio as a function of the ROI size. In this scenario, the reduced amount of data to be transferred through the slip ring (as the number of pixels is reduced by the "binning") can be used to increase/adapt the number of projections to achieve the desired isotropy measure *τ*.

In another embodiment, different reconstructions are performed for the entire volume at different scan FOVs. For some or each of these reconstructions, different detector binning setups g may be used. The resulting (sub-)images *mₖ* may then be combined (stitched) into a single image overall "super" image *m* = U*ₖmₖ*. Optionally, transition(s) between sub-images may be smoothed by filtering. In this scenario, the result with the strongest binning preferably contributes to the outer region of the FOV, while the result with the weakest binning preferably contributes to the inner region. The combination can be done by a post-reconstruction image processing component (not shown) of the facilitator system FS or of the computing system. The described combination is of general application herein where the adjusted imaging parameters are obtained for the respective sub-images/ sub-FOV, one set of such adjusted imaging parameters per sub-images/ sub-FOV and the sub-images/ sub-FOV are then combined into the (final) image *m.*

### Adjusting p as filtering (either in image domain or projection domain), given an isotropy measure τ acceptability range:-

Local anisotropy may mean that a better resolution in radial direction can be observed than in azimuthal direction, or the other way around. Thus, isotropy can be established by a reduction of resolution in radial direction *r* or in azimuthal direction *r*^{⊥}. Taking as an example the reduction of resolution in radial direction, this reduction is preferably stronger with increasing distance to the rotation axis/iso center c.

This selective reduction of resolution along either of the reference direction may be obtained either by applying a filter *f* in image domain, or by applying such filter *g* in the projection domain. For filtering in image domain, a Gaussian filter applied in, says, radial direction may be used. Filter parameters of this filter may be configured to change as a function of distance to the rotation axis. These parameters can be determined experimentally for various acquisition schemes (remaining imaging parameters). For filtering in projection domain, one may use a Gaussian filter along detector rows. Parameters *p* of this filter may need to change as a function of distance to the detector center. These parameters can be determined experimentally for various acquisition schemes (settings of remaining p's).

The filtering/deconvolution can also be applied after re-binning to wedge/parallel beam geometry when the data is sorted and interpolated on a regular grid on the *r-φ* space.

### Adjusting p as focal spot size, given imaging requirement q a fixed number of views per rotation, and given an isotropy measure τ acceptability range:-

Depending on the selected combination of the axial FOV and the matrix size in image domain (as per operation of grid definer GD), the maximal possible image resolution may not be achievable in the image center, and the selection of a larger focal spot is not necessary. As smaller focal spot size may be adjusted for, still meeting the isotropy measure *τ* acceptability range, without a reduction of resolution in the iso-center.

The above scenarios are merely illustrations of various combinations of adjustable imaging parameters and imaging requirements. Others are also envisaged. Also, it will be understood that some of the above described techniques such as selective filtering along only one of the reference directions for reducing resolution along the selected reduction for ensuring *τ* as a ratio is within *Iₐ*, or the reconstruction into sub-images and combination into super image, etc., are not tied to the above mentioned scenarios, but may also be used in other scenarios/applications, and such is specifically envisaged herein in various embodiments.

Components of facilitator system FS may be implemented as one or more software modules, run on one or more computing nodes associated with the imager IA. The system FCS may be integrated at least in part in the operating console, or indeed partly or fully into the detector module XD.

Alternatively, some or all components of the system FS, in particular the IQ assessor IQA, may be arranged in hardware such as a suitably programmed microcontroller or microprocessor, such as an FPGA (field-programmable-gate-array) or as a hardwired IC chip, an application-specific integrated circuitry (ASIC), integrated into the imager IA in particular its detector XD. In a further embodiment still, the system FCS may be implemented in both, partly in software and partly in hardware.

The different components of the system FS may be implemented on a single computing unit. Alternatively, some or more components are implemented on different computing units, possibly remotely arranged in a distributed architecture and connectable in a suitable communication network.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

**In** the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs (indicated herein in between brackets "( )" ) in the claims laid out in the section "CLAIMS" below should not be construed as limiting the scope.

## Claims

1. System for facilitating operation of a rotational tomographic X-ray imaging apparatus (IA), comprising: -
a user input interface (USI) capable of allowing a user of the imaging apparatus to specify desired one or more imaging requirements as user input; and
an adjuster (AD) configured to adjust, based on the user input, one or more imaging parameters in respect of the imaging apparatus so as to improve isotropy of spatial resolution in tomographic imagery obtainable by operation of the imaging apparatus (IA) at the adjusted parameters.

2. System of claim 1, wherein the adjuster (AD) is to so adjust one or more imaging parameters based on an isotropy measure.

3. System of claim 2, wherein the adjuster (AD) is to so adjust one or more imaging parameters such that the isotropy measure remains within an acceptability range.

4. System of any one of preceding claims, wherein said isotropy measure is based on respective spatial resolution along plural reference directions in image domain.

5. System of any one of preceding claims, wherein said isotropy measure is based on a ratio of such spatial resolutions, including one such spatial resolution along a radial direction in respect of an acquisition rotation of the imaging apparatus, and spatial resolution along a direction perpendicular to such radial direction.

6. System of any one of preceding claims, wherein said imaging parameters so adjustable pertain to any one or more of: i) frame number per rotation, ii) rotation period, iii) filtering in projection domain or image domain, iv) focal spot size.

7. System of any one of preceding claims, wherein the said adjusting by adjuster (AD) is based on calibration data measured in relation to the, or a, similar imaging apparatus (IA).

8. System of any one of preceding claims, wherein the system, if no such improvement in isotropy is achievable given the user input, to issue a notification.

9. System of any one of preceding claims, when the system, on receipt of an acceptance signal, or automatically, to forward such adjusted one or more imaging parameters to control interface (CI) circuitry of imaging apparatus to thereby cause applying the imaging parameters to one or more components (CIM) of the imaging apparatus, thereby readying the imaging apparatus for imaging.

10. System of any one of preceding claims, wherein the said one or more imaging requirements include one or more of: i) distance to iso-center, ii) level of spatial and/or temporal resolution.

11. Imaging arrangement (IAR), including a system as per any one of the preceding claims, and including any one or more of: i) the imaging apparatus, ii) a display device on which the user interface is presented, iii) a storage medium on which is stored data on which the adjusting is based, iv) a reconstructor for reconstructing projection data acquirable at the imaging parameters into tomographic imagery.

12. Method of facilitating operation of a rotational tomographic X-ray imaging apparatus, comprising: -
receiving (S530), via a user input interface (USI) user input including one or more imaging requirements in respect of an imaging operation of the imaging apparatus;
adjusting (S540), based on the user input, one or more imaging parameters in respect of the imaging apparatus so as to improve isotropy of spatial resolution in tomographic imagery obtainable by operation of the imaging apparatus (IA) at the adjusted parameters.

13. Method of measuring and storing such calibration data as in claim 7.

14. A computer program element, which, when being executed by at least one processing system, is adapted to cause the processing system to perform the method as per claim 12 or 13.

15. At least one computer readable medium having stored thereon the program element of claim 14, or having stored thereon the calibration data of claim 13.
